Europäisches Patentamt

(19) European Patent Office

Office européen des brevets

(11) Numéro de publication: **0 310 531**
A1

(12) # DEMANDE DE BREVET EUROPEEN

(21) Numéro de dépôt: **88430023.7**

(22) Date de dépôt: **02.09.88**

(51) Int. Cl.⁴: **A 61 B 5/10**
A 61 F 5/14

(30) Priorité: **02.10.87 FR 8713818**

(43) Date de publication de la demande:
**05.04.89 Bulletin 89/14**

(84) Etats contractants désignés:
**BE CH DE ES GB IT LI SE**

(71) Demandeur: **Poussou, Alain A.**
**Les Myosotis -Bâtiment B 190, Avenue des Caillols**
**F-13012 Marseille (FR)**

(72) Inventeur: **Poussou, Alain A.**
**Les Myosotis -Bâtiment B 190, Avenue des Caillols**
**F-13012 Marseille (FR)**

(74) Mandataire: **Azais, Henri et al**
**c/o CABINET BEAU DE LOMENIE 14, rue Raphael**
**F-13008 Marseille (FR)**

(54) **Procédés et dispositifs pour obtenir des empreintes corrigées des pieds afin de fabriquer des semelles correctrices.**

(57) L'invention a pour objet des procédés et des dispositifs pour obtenir des empreintes corrigées des pieds afin de fabriquer des semelles correctrices.

Un dispositif selon l'invention comporte trois cadres rigides superposés (4, 5 et 6). Chaque cadre est équipé d'une paroi flexible supérieure (4a, 5a, 6a) et inférieure (4b, 5b, 6b) qui délimitent avec ledit cadre une enceinte étanche (4c, 5c, 6c) remplie d'un matériau granuleux, laquelle enceinte communique avec un appareil d'aspiration (7) par une canalisation flexible (4d, 5d, 6d) munie d'une vanne (4e, 5e, 6e). Les trois cadres sont reliés à un bâti fixe (3) par des bras (4g, 5g, 6g) de longueur différente articulés audit bâti.

Une application est la fabrication de tout type de semelles correctrices.

Fig.1

EP 0 310 531 A1

## Description

## Procédés et dispositifs pour obtenir des empreintes corrigées des pieds afin de fabriquer des semelles correctrices.

La présente invention a pour objet des procédés et des dispositifs destinés à obtenir des empreintes corrigées des pieds afin de fabriquer des semelles correctrices ou orthèses plantaires ou des chaussures comportant une semelle correctrice.

Le secteur technique de l'invention est celui de la fabrication des semelles et chaussures orthopédiques.

On connaît des procédés permettant d'obtenir une empreinte négative ou complémentaire de la plante des pieds au moyen d'une enceinte déformable et étanche, par exemple une poche déformable, qui est remplie de matériaux granuleux et qui est mise en dépression après qu'un pied a été posé sur la paroi déformable qui constitue la face supérieure de l'enceinte.

Le brevet U.S. A. 2.472.754 (W.J. MEAD) décrit un procédé selon lequel on place un pied dont on désire obtenir un moulage dans un conteneur rigide comportant un fond et des parois latérales et contenant plusieurs poches étanches et flexibles, qui sont remplies d'un matériau granuleux mélangé à un fluide. Une des poches est placée dans le fond du conteneur, sous la plante du pied posé à plat. Les autres poches sont placées autour et au-dessus du pied. Ces poches sont reliées à un moyen d'aspiration du fluide.

Après enlèvement du pied, on replace les poches dans leur position et on fabrique un moulage du pied dans la cavité. Ce moulage est utilisé comme modèle pour fabriquer une forme à chaussures reproduisant la forme du pied. On peut également obtenir un modèle de la cavité en insérant dans celle-ci une poche flexible que l'on gonfle, que l'on remplit d'un mélange d'un matériau granuleux et d'un fluide et que l'on fige en mettant la poche en dépression.

Le dispositif décrit peut être utilisé par les vendeurs de chaussures pour choisir des chaussures adaptées aux pieds d'un client.

La publication FR. A. 2.162.020 (L> HAGGLUND) décrit des procédés pour fabriquer des moulages ayant une forme qui correspond à une partie du corps d'un patient.

Selon ces procédés, on utilise un moule étanche et souple comportant ou moins une paroi souple qui est appliquée contre une partie du corps, lequel moule contient une matière granuleuse et des moyens pour le mettre en dépression. Il est prévu que l'on peut utiliser le procédé pour fabriquer des chaussures orthopédiques, des semelles, des supports de voûte plantaire sans donner aucune description détaillée du procédé utilisé dans ce cas.

Le brevet DE. C. 1.170.114 (B. HULLMANN) décrit un procédé et des dispositifs pour obtenir une empreinte du pied posé à plat selon lesquels on appuie le pied sur une membrane flexible, qui constitue la paroi supérieure d'un récipient rempli de billes qui est mis ensuite en dépression par aspiration à travers une paroi perméable.

Le brevet DE. C. 1.234.355 (B. HULLMANN) décrit des dispositifs du même type que les précédents qui comportent, en outre, un couvercle composé d'un cadre portant une membrane déformable qui est appliquée par la dépression contre une matière modelable, qui est placée entre les deux parois déformables pour obtenir un élément orthopédique qui reproduit la forme de la plante du pied.

Tous ces brevets antérieurs décrivent des procédés qui permettent d'obtenir une empreinte négative de la plante ou de la totalité du pied au moyen d'une enceinte remplie d'un matériau granuleux et comportant au moins une paroi supérieure souple, sur laquelle s'appuie la plante du pied avant qu'on ne mette l'enceinte en dépression.

Dès lors que l'on a obtenu une empreinte négative constituée par la face supérieure flexible d'une enceinte remplie d'un matériau granuleux et maintenue en dépression, il est possible d'apporter à cette empreinte des corrections en creux en appuyant localement sur la paroi flexible pour repousser le matériau granuleux. Par contre, il est difficile d'apporter à cette empreinte négative des corrections en relief ou d'obtenir une empreinte de la plante du pied placée dans une position différente de la position à plat, afin de fabriquer des semelles ou des éléments de semelles correcteurs. De plus, si les corrections en creux sont effectuées sur une enceinte qui est isolée du moyen de mise en dépression, il peut se former des bulles ou des plis.

L'objectif de la présente invention est de procurer des moyens qui permettent de corriger les empreintes négatives aussi bien en relief qu'en creux en évitant la formation de bulles ou de plis et qui permettent également d'obtenir des empreintes négatives et positives qui reproduisent la forme de la plante du pied placé dans une position déterminée, de sorte que l'on peut fabriquer ensuite, à partir de ces empreintes négatives et positives corrigées, des semelles correctrices ou des éléments de semelle correcteurs de défauts.

Un procédé selon l'invention pour obtenir une empreinte négative d'un pied est du type selon lequel on appuie le pied sur la paroi supérieure flexible d'une première enceinte étanche, qui est remplie de matériaux granuleux, qui comporte une paroi supérieure et une paroi inférieure flexibles et qui est reliée à un appareil d'aspiration permettant de la mettre en dépression pour rigidifier lesdits matériaux granuleux et pour conserver une empreinte négative du pied.

Les objectifs de l'invention sont atteints par un procédé selon lequel on corrige ladite empreinte négative pendant que ladite enceinte est connectée audit appareil d'aspiration qui aspire dans ladite enceinte.

Avantageusement, on obtient, en outre, une empreinte positive complémentaire de l'empreinte négative corrigée en posant sur celle-ci une deuxième enceinte étanche qui est remplie de matériaux granuleux, qui comporte une paroi supérieure et une paroi inférieure flexibles et qui est reliée à un appareil d'aspiration permettant de la mettre en dépression et en posant ledit pied sur ladite paroi supérieure et on fabrique une semelle correctrice ou un élément de semelle correcteur en utilisant ladite empreinte positive seule ou en combinaison avec la dite empreinte négative corrigée.

Avantageusement, on corrige localement en creux ladite empreinte positive en relevant ladite deuxième enceinte et en appuyant localement sur la paroi inférieure de celle-ci pendant que l'enceinte est connectée audit moyen d'aspiration, on place à nouveau ladite deuxième enceinte sur la première enceinte et on corrige ladite empreinte négative en appuyant ladite empreinte positive corrigée contre la face supérieure de ladite première enceinte pendant que celle-ci est connectée audit moyen d'aspiration et on fabrique une semelle correctrice ou un élément de semelle correcteur en utilisant ladite empreinte positive corrigée seule ou en combinaison avec ladite empreinte négative corrigée.

Avantageusement, on obtient une première empreinte négative corrigée en plaçant des éléments correcteurs sous la paroi inférieure d'une enceinte inférieure puis en appuyant le pied sur la paroi supérieure de ladite enceinte inférieure.

Selon un mode de réalisation préférentiel, on obtient une empreinte négative corrigée par la suite d'opérations suivantes :
- on prend une première empreinte négative de la plante du pied en appuyant celui-ci sur la paroi supérieure souple d'une enceinte inférieure remplie d'un matériau granuleux que l'on met ensuite en dépression;
- on corrige éventuellement ladite première empreinte;
- on pose une deuxième enceinte comportant une paroi supérieure et une paroi inférieure flexibles sur ladite empreinte négative, on appuie à nouveau le pied sur la paroi supérieure de ladite deuxième enceinte et on met ensuite celle-ci en dépression, de sorte que ladite paroi supérieure de la deuxième enceinte conserve une empreinte négative de la plante du pied qui est corrigée par la présence de ladite première empreinte négative.

Avantageusement, on apporte des corrections locales en relief aux empreintes négatives en relevant lesdites enceintes et en appuyant localement sur la paroi flexible inférieure de ladite enceinte inférieure ou de ladite deuxième enceinte pendant que celle-ci est connectée sur ledit moyen de mise en dépression.

Un dispositif selon l'invention est du type comportant au moins une enceinte étanche ayant au moins une paroi flexible qui est remplie d'un matériau granuleux et qui peut être connectée sur un appareil d'aspiration par l'intermédiaire d'une vanne.

Un dispositif selon l'invention est caractérisé par le fait qu'il comporte au moins deux cadres horizontaux, rigides et superposés qui sont équipés chacun d'une paroi supérieure et d'une paroi inférieure flexibles qui délimitent avec ledit cadre une enceinte étanche qui est reliée à un appareil d'aspiration par une canalisation flexible munie d'une vanne et qui est remplie d'un matériau granuleux.

Selon un mode de réalisation préférentiel, un dispositif selon l'invention comporte un troisième cadre rigide et amovible qui est superposable au deuxième cadre, qui est équipé d'une paroi supérieure et d'une paroi inférieure flexibles, qui délimitent avec ledit troisième cadre une enceinte étanche qui est remplie d'un matériau granuleux et qui est connectée à un appareil d'aspiration par une canalisation flexible équipée d'une vanne.

L'invention a pour résultat des dispositifs qui permettent d'obtenir une empreinte négative du pied, de corriger celle-ci, d'obtenir ensuite une empreinte positive complémentaire de l'empreinte négative corrigée, de corriger localement l'empreinte positive, d'obtenir une nouvelle empreinte négative complémentaire de l'empreinte positive corrigée afin de fabriquer des semelles correctrices qui ne reproduisent pas seulement la forme du pied, mais qui tendent à corriger la posture de celui-ci.

Les dispositifs selon l'invention permettent d'apporter de nombreuses corrections.

Ils permettent en premier lieu de prendre une empreinte négative d'un pied placé dans une position donnée en insérant des éléments correcteurs sous la paroi inférieure de l'enceinte inférieure qui est flexible, de sorte que ces éléments correcteurs peuvent exercer une influence sur la posture du pied.

Ils permettent également d'apporter des corrections locales sur une empreinte négative et non seulement des corrections en creux obtenues en appuyant localement sur la face supérieure qui conserve ladite empreinte négative, afin de repousser le matériau granuleux, mais également des corrections en relief de la face supérieure. Ces corrections en relief sont obtenues en relevant les enceintes pour accéder aux faces inférieures flexibles et en appuyant localement sur celles-ci pour déplacer les matériaux granuleux vers la face supérieure.

Les dispositifs selon l'invention permettent d'adoucir la courbure des corrections locales d'une première empreinte négative en obtenant une deuxième empreinte négative de la première empreinte négative après correction de celle-ci.

Les dispositifs selon l'invention permettent également d'obtenir une empreinte positive complémentaire d'une empreinte négative corrigée, ce qui permet d'apporter des corrections locales en creux sur l'empreinte positive puis de reproduire ces corrections en relief sur une empreinte négative complémentaire de l'empreinte positive corrigée.

Toutes ces corrections peuvent être obtenues en intervenant sur les parois flexibles des enceintes pendant que ces dernières sont en liaison avec l'appareil d'aspiration, ce qui facilite les déplacements des matériaux granuleux et évite la formation de plis ou de bulles.

Les procédés et dispositifs selon l'invention permettent d'obtenir, de façon simple, rapide et économique, de nombreuses possibilités de correction de l'empreinte initiale pour aboutir à une semelle ou à une chaussure correctrice.

Ils permettent d'obtenir un meilleur contrôle de la largeur des empreintes en vue d'une meilleure adaptation de la semelle correctrice dans un type de chaussure défini.

Ils permettent d'obtenir des empreintes enveloppantes qui remontent sur les côtés du pied, ce qui permet de réaliser des orthèses présentant un meilleur maintien latéral et arrière du pied qui sont plus efficaces.

La description suivante se réfère aux dessins annexés qui représentent, sans aucun caractère limitatif, un exemple de réalisation d'un dispositif selon l'invention et les étapes de différents procédés selon l'invention.

La figure 1 est une coupe verticale d'un mode de réalisation préférentiel d'un dispositif selon l'invention.

Les figures 2 à 7 représentent, en coupes verticales, des étapes successives des procédés selon l'invention utilisant un dispositif selon la figure 1.

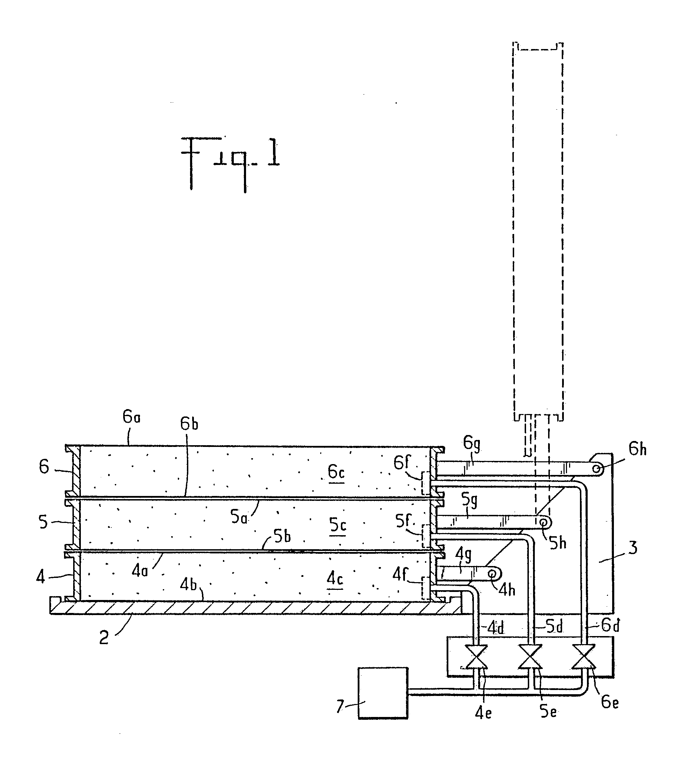

La figure 1 représente un dispositif qui comporte un bâti fixe, qui est composé avantageusement d'une embase plane 2 posée sur une surface horizontale plane et de deux montants verticaux 3.

Il comporte trois cadres rectangulaires identiques superposés 4, 5 et 6 composés par exemple de profilés en U.

Ces cadres rigides ont une longueur supérieure à la longueur des plus grands pieds et une largeur supérieure à la largeur habituelle d'un pied ou de deux pieds, de telle sorte que l'on puisse placer un ou deux pieds à l'intérieur desdits cadres. Chacun des cadres porte deux parois flexibles déformables, une paroi supérieure 4a, 5a, 6a et une paroi inférieure 4b, 5b, 6b, qui sont constitués par exemple par des feuilles de matière plastique souple tendues en travers du cadre.

Ces parois sont assemblées de façon étanche avec le cadre correspondant, de sorte que chaque cadre et les deux parois flexibles qui l'équipent délimitent une enceinte 4c, 5c, 6c qui est remplie d'un matériau granuleux, par exemple de microbilles de verre ou de matière plastique ayant un diamètre de l'ordre du millimètre.

En variante, les deux parois flexibles équipant chaque cadre peuvent être remplacées par une poche déformable, par exemple par un sac en matières plastique fixé sur le périmètre du cadre, de préférence de telle sorte que la paroi supérieure soit tendue.

La hauteur des cadres 4, 5 et 6 est de l'ordre de 2 à 12 centimètres.

Chaque enceinte 4c, 5c, 6c est connectée par une petite canalisation flexible 4d, 5d, 6d sur un appareil d'aspiration 7, qui est par exemple une pompe à vide en passant par un robinet ou une vanne 4e, 5e, 6e, qui permet d'isoler l'enceinte de l'appareil 7. L'appareil 7 aspire l'air contenu dans l'enceinte, de sorte qu'il crée une dépression à l'intérieur de celle-ci, qui permet de figer les particules qu'elle contient dans une configuration donnée, ce qui permet de conserver une empreinte de façon connue.

L'extrémité de chaque canalisation 4d, 5d, 6d qui débouche respectivement dans les enceintes 4c, 5c, 6c est équipée d'un filtre 4f, 5f, 6f qui évite l'aspiration par la pompe 7 des particules contenues dans l'enceinte.

Il est précisé que l'embase 2 n'est pas obligatoire. En variante, la cadre inférieur 4 peut être posé directement sur un sol horizontal.

La figure 1 représente un mode de réalisation dans lequel chaque cadre 4, 5 et 6 est relié par un ou plusieurs bras 4g, 5g, 6g à un axe d'articulation horizontal 4h, 5h, 6h porté par les deux montants 3, de sorte qu'il est possible de relever chaque cadre à la verticale pour accéder à la paroi flexible inférieure pendant que l'enceinte correspondante est en communication avec l'appareil d'aspiration 7.

A titre d'illustration, on a représenté en pointillés sur la figure 1 le cadre 5, en position relevée. Les axes d'articulation 4h, 5h, 6h sont disposés dans un plan transversal incliné à 45° sur l'horizontale pour permettre de relever les cadres 4, 5 et 6 côte à côte.

Du fait que les cadres sont reliés par des bras à des articulations fixées au bâti, ils se retrouvent automatiquement superposés lorsqu'on les rabat à l'horizontale.

Bien entendu, on pourrait arriver au même résultat par d'autres moyens mécaniques, par exemple en faisant coulisser les cadres 4, 5 et 6 sur des tiges de guidage ou entre des glissières verticales.

La figure 1 représente un exemple préférentiel dans lequel les trois cadres 4, 5 et 6 sont identiques et superposables.

Selon une variante, le cadre supérieur 6 peut être plus petit et il peut ne pas être relié au bâti fixe.

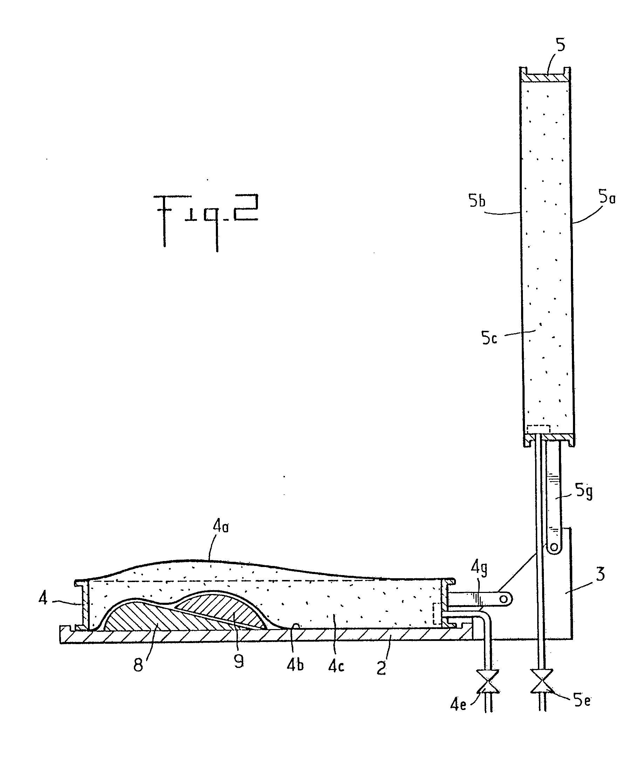

La figure 2 représente en coupe verticale une première étape d'un procédé selon l'invention mettant en oeuvre un dispositif selon la figure 1 qui comporte seulement deux cadres 4 et 5 reliés à un bâti 2, 3.

Dans cette première étape, le cadre 5 est relevé. On place sous la paroi inférieure 4b de l'enceinte 4c des éléments de correction destinés à forcer le pied à prendre une posture déterminée lorsque l'on prendra l'empreinte négative de la plante du pied.

Ces éléments correcteurs comportent par exemple une talonnette 8 qui va se trouver sous le talon pour obliger le pied à s'incliner vers l'avant dans une position analogue à celle qu'il occupe dans une chaussure munie de talons relativement hauts. Un autre élément correcteur qui peut être utilisé seul ou en combinaison avec une talonnette est un correcteur de voûte plantaire 9 qui oblige le pied à prendre une position cambrée. On peut placer ainsi sous la paroi inférieure 4b des gabarits de calage ou d'autres éléments précorrecteurs.

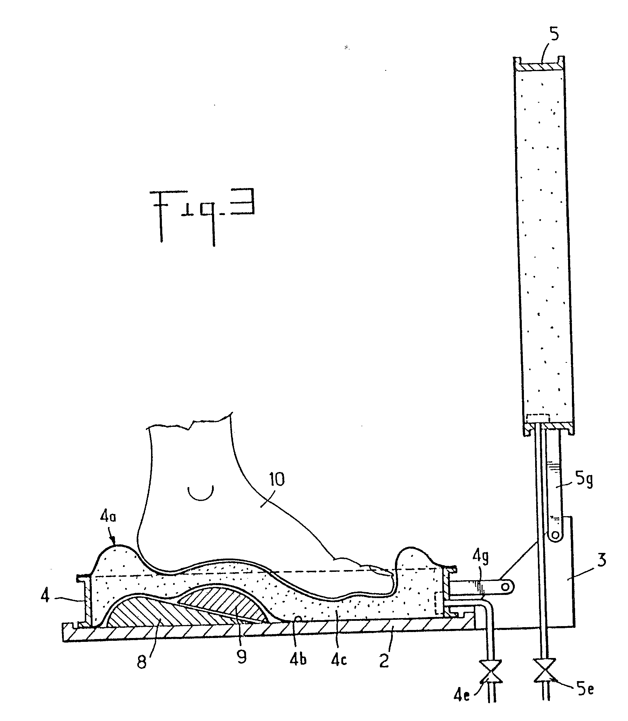

La figure 3 est une coupe verticale analogue à la figure 2, qui représente une étape suivante du procédé dans laquelle on appuie le pied 10 du patient sur la paroi supérieure 4a, de sorte que le matériau granuleux contenu dans l'enceinte 4c se déplace et que le paroi 4a épouse la forme de la plante du pied et on met l'enceinte 4c en dépression en ouvrant la vanne 4e qui la met en communication avec l'appareil d'aspiration 7, ce qui a pour effet de rigidifier les particules solides, de sorte que la paroi 4a est figée dans une forme qui constitue une empreinte négative de la plante du pied 10.

Il est précisé que le terme empreinte négative est utilisé pour désigner une empreinte complémentaire dans laquelle les creux du pied sont remplacés par des bosses en relief et les reliefs par des parties en creux. Le terme empreinte positive est utilisé pour désigner une empreinte qui reproduit directement la forme du pied.

L'empreinte négative que l'on obtient ne reproduit pas simplement la forme du pied posé à plat. C'est une empreinte qui est corrigée par le fait que l'on a placé des éléments correcteurs sous la paroi flexible 4b et que grâce à la déformabilité de celle-ci et à la faible épaisseur de l'enceinte 4c ces éléments correcteurs ont forcé le pied à prendre une position déterminée pendant la prise d'empreinte.

Les figures 2 et 3 montrent donc une première possibilité offerte par les dispositifs selon l'invention pour corriger l'empreinte négative d'un pied afin d'obtenir ensuite une semelle correctrice. On remarquera que cette première correction de l'empreinte négative est réalisée pendant que l'on aspire dans la cavité 4c au moyen de l'appareil 7, ce qui a pour effet de faciliter l'influence que les éléments correcteurs 8, 9 placés sous la paroi 4b exercent sur la position du pied.

Lorsque le pied 10 est appuyé sur la paroi flexible 4a, les particules situées sous le pied tendent à migrer vers l'extérieur, hors de la zone qui est soumise à la pression du pied, de sorte qu'à la fin de l'étape représentée sur la figure 3, l'épaisseur de l'enceinte 4c est faible sous le pied et la paroi 4a remonte à l'avant, à l'arrière et sur les côtés du pied et on obtient une empreinte négative qui comporte des bords relevés qui enveloppent légèrement l'empreinte corrigée de la plante du pied. Le fait que l'épaisseur de l'enceinte 4c soit très faible sous la plante du pied fait que celle-ci épouse sensiblement le contour des éléments correcteurs 8 et 9. Ce résultat ne pourrait pas être atteint avec une enceinte 4c dont la paroi inférieure 4b ne serait pas flexible;

Une fois que l'on a pris l'empreinte négative corrigée de la face inférieure du pied, on peut encore apporter à cette empreinte des corrections en appuyant localement sur la paroi 4a pour repousser localement le matériau en grains afin d'apporter des corrections en creux.

Grâce à un dispositif selon l'invention, on peut également faire sur l'empreinte négative des corrections en relief localisées.

Pour cela, après avoir enlevé le pied 10, on soulève le cadre 4, ce qui permet d'accéder à la paroi flexible inférieure 4b et on peut alors repousser localement cette paroi, soit manuellement, soit avec un outil pour déplacer le matériau en grains contenu dans l'enceinte 4c, de telle sorte qu'il repousse localement la paroi supérieure 4a vers l'extérieur en corrigeant la forme de celle-ci.

Ces corrections localisées en creux ou en relief de l'empreinte négative sont exécutées pendant que l'enceinte 4c est reliée à l'appareil 7, c'est-à-dire pendant que la vanne 4e est ouverte, de telle sorte que l'aspiration facilite le déplacement des particules solides à l'intérieur de l'enceinte et permet d'apporter des corrections localisées sans aucun risque de formation de plis sur la paroi 4a ou de bulles d'air dans l'enceinte 4c.

Le modelage localisé en creux ou en relief de la paroi 4 a constitue une deuxième possibilité offerte par les dispositifs selon l'invention pour apporter des corrections à l'empreinte négative de la plante d'un pied afin d'obtenir ultérieurement une semelle correctrice.

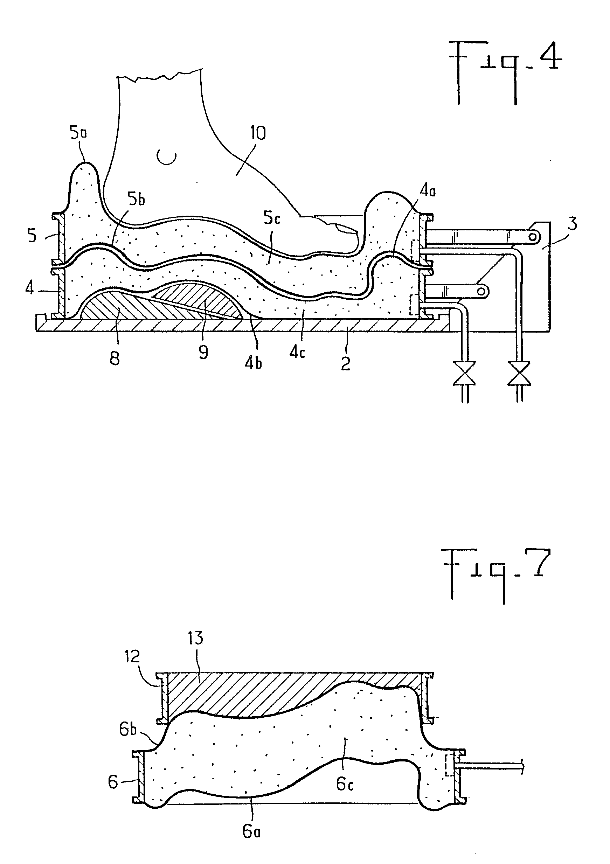

La figure 4 représente, en coupe verticale, une étape suivante d'un procédé selon l'invention.

On retrouve sur cette figure les deux cadres 4 et 5 superposés.

L'enceinte 4c est en dépression et la paroi supérieure 4a forme une première empreinte négative et corrigée de la plante du pied 10.

Après avoir descendu le cadre 5, ce qui amène l'enceinte 5c au-dessus de l'empreinte négative 4a, on appuie à nouveau le pied 10 sur la paroi supérieure 5a de la deuxième enceinte pendant que celle-ci est mise en communication avec l'appareil d'aspiration 7, de sorte que l'on obtient une deuxième empreinte négative formée par la paroi 5a.

Au cours de cette opération, la première empreinte négative 4a qui a été déjà corrigée joue le rôle que jouaient les éléments 8 et 9 lors de la prise de la première empreinte.

Elle s'appuie contre la paroi flexible 5b et déforme celle-ci, ce qui a pour effet de repousser les matériaux granuleux et d'obliger le pied 10 à occuper une position corrigée par rapport à la position naturelle.

Les corrections qui ont été apportées à la première empreinte 4a sont répercutées sur la deuxième empreinte négative 5a, mais sous une forme atténuée, ce qui permet d'adoucir les formes et les pentes des corrections qui seraient trop accentuées. De plus, les matériaux granuleux contenus dans l'enceinte 5c sont repoussés sur les côtés du pied qui sont déjà occupés par les bords relevés de la première empreinte 4a, de sorte que l'on obtient ainsi une deuxième empreinte négative qui est encore corrigée par rapport à la première et qui est plus enveloppante, c'est-à-dire dont les bords sont relevés plus haut à l'avant, à l'arrière et sur les côtés de la plante du pied sans rien perdre de la précision de l'empreinte négative corrigée..

On peut évidemment apporter, si nécessaire, soit manuellement, soit avec un outil des retouches locales en creux ou en relief à cette deuxième empreinte négative 5a comme on l'a fait pour la première pendant que l'enceinte 5c est reliée à l'appareil d'aspiration.

Pendant l'opération représentée sur la figure 4, la paroi inférieure 5b de la deuxième enceinte 5c épouse la forme de la paroi 4a et après mise en dépression de l'enceinte 5c, elle conserve sa forme qui constitue une empreinte positive de l'empreinte négative corrigée 4a.

On peut accessoirement utiliser cette empreinte positive pour former une semelle correctrice par exemple en plaçant entre les parois 4a et 5b une plaque de matière thermoformable chaude, qui épouse la forme corrigée et qui constitue une semelle correctrice.

On peut également retourner l'enceinte 5c de façon à placer l'empreinte positive 5b sur le dessus et couler dans celle-ci une matière durcissable, par exemple une résine polymérisable qui se transforme en durcissant en une semelle correctrice épaisse dont la face placée au contact de la paroi 5b constituera la face supérieure correctrice qui sera placée au contact de la plante du pied.

Afin d'utiliser au mieux les possibilités de corrections offertes par la deuxième empreinte négative 5a, on utilise, de préférence, une deuxième empreinte positive qui reproduit la forme de la deuxième empreinte négative corrigée 5a.

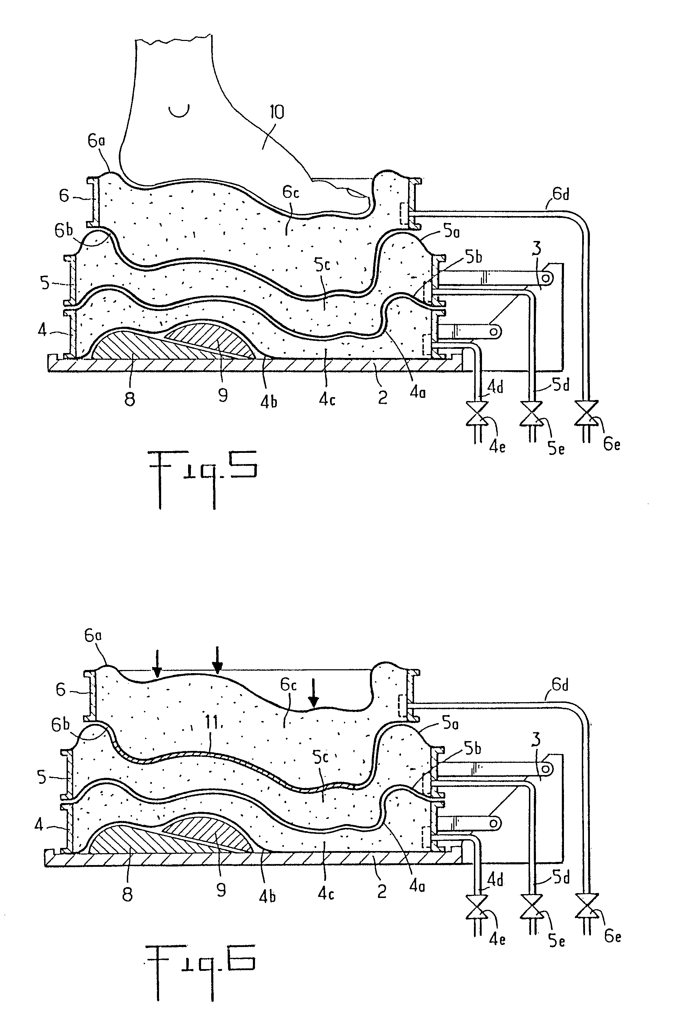

La figure 5 représente une étape suivante d'un procédé selon l'invention au cours de laquelle on obtient une empreinte positive complémentaire de la deuxième empreinte négative 5a.

On retrouve sur la figure 5 le cadre inférieur 4 équipé de deux parois flexibles 4a, 4b dont la paroi supérieure 4a garde une première empreinte négative corrigée et un deuxième cadre 5 équipé de deux parois flexibles 5a, 5b dont la paroi supérieure 5a reproduit une deuxième empreinte négative et corrigée de la plante du pied.

On utilise un troisième cadre rigide 6 équipé de deux parois flexibles 6a, 6b qui délimitent une enceinte 6c, remplie d'un matériau à l'état divisé, qui peut être mise en communication avec un appareil d'aspiration par une canalisation flexible 6d.

La figure 5 représente un exemple dans lequel le cadre 6 est plus petit que les cadres 4 et 5 et n'est pas articulé sur le bâti fixe 3. En variante, le cadre 6 peut être identique aux cadres 4 et 5 et articulé au bâti 3 comme le représente la figure 1.

Une fois le cadre 6 mis en place, on demande au patient d'appuyer son pied 10 sur la paroi supérieure 6a, de telle sorte que la paroi inférieure 6b épouse la forme de la paroi 5a au contact de laquelle elle se trouve et on ouvre le robinet 6e qui met l'enceinte 6c en communication avec l'appareil d'aspiration 7.

Au cours de cette étape on peut remplacer la pression du pied 10 par celle d'une forme standard de pied.

Une fois que l'on a pris l'empreinte positive du pied, on peut corriger celle-ci localement en creux. Pour cela, on enlève le pied et on soulève le cadre 6 pour avoir accès à la paroi 6b tout en laissant le robinet 6e ouvert et on appuie localement sur la paroi 6b pour repousser localement les matériaux contenus dans l'enceinte 6c.

Cette possibilité d'apporter localement des corrections en creux à l'empreinte positive formée par la paroi 6b permet d'obtenir des corrections en relief de l'empreinte négative 5a.

Une fois l'empreinte positive 6b corrigée, on remet le cadre 6 sur le cadre 5 et on met à nouveau l'enceinte 5c en communication avec l'appareil 7, de sorte que la paroi supérieure 5a se déforme et épouse la forme de la paroi 6b, c'est-à-dire une forme qui constitue une empreinte négative à laquelle on a apporté des corrections locales en relief qui reproduisent les corrections en creux apportées à la paroi 6b.

On peut alors utiliser l'empreinte négative corrigée 5 a et l'empreinte positive 6b qui sont complémentaires pour fabriquer une semelle orthopédique correctrice comme le montre la figure 6.

Au cours de cette étape, on place une feuille thermoplastique 11 entre la paroi 5a et la paroi 6b, les enceintes 5c et 6c étant en dépression, de sorte qu'elles sont indéformables et on exerce une pression sur la paroi 6a, de sorte que la feuille 11 constitue, après durcissement, une semelle correctrice que l'on peut placer à l'intérieur d'une chaussure.

La figure 6 montre que cette semelle correctrice peut remonter à l'arrière du talon. Elle peut également remonter sur les côtés latéraux du pied, de sorte qu'elle confère un maintien plus efficace du pied dans la position correctrice recherchée.

La figure 7 représente une variante d'utilisation de l'empreinte positive corrigée 6b.

Selon cette variante, on retourne le cadre 6, de telle sorte que la paroi 6b qui constitue l'empreinte positive corrigée, soit au-dessus.

On place autour de la partie centrale de l'enceinte positive 6b un coffrage étanche 12, dont le fond est constitué par la paroi 6b et on verse dans celui-ci un matériau liquide durcissable par exemple un latex ou une résine polymérisable. Après durcissement, on obtient une semelle correctrice épaisse 13 ayant une face supérieure sensiblement horizontale et une face inférieure qui épouse le contour de la paroi 6b. On retourne cette semelle et on la place à l'intérieur d'une chaussure.

Un dispositif selon l'invention permet de fabriquer des semelles dites proprioceptives, qui comportent des éléments en relief placés à des endroits bien déterminés pour exciter certains muscles et provoquer ainsi une correction automatique de la position du pied.

. Pour obtenir des semelles comportant ces éléments correctifs en relief, il suffit d'apporter dans l'empreinte négative 4a ou 5a, des corrections en creux bien localisées, qui donneront naissance à des éléments en relief sur la semelle correctrice.

Un appareil selon l'invention permet également de reproduire des semelles ou des éléments de semelle ou de réaliser des assemblages de plusieurs éléments de semelle en utilisant deux enceintes superposées.

On place une plaque plane entre les deux enceintes et l'on met en dépression une des deux enceintes, par exemple l'enceinte inférieure pendant qu'elles sont rapprochées l'une de l'autre.

On soulève l'enceinte supérieure, on enlève la plaque, on imprime en creux les éléments à reproduire sur la paroi supérieure de l'enceinte inférieure, on coule ensuite un liquide durcissable sur la paroi inférieure, qui donne naissance à une semelle correctrice ou proprioceptive.

On peut également prendre une empreinte positive complémentaire en appuyant la deuxième enceinte que l'on met en dépression, puis on place entre les deux enceintes une plaque thermoformable et on rapproche les deux enceintes pour obtenir une semelle réceptrice ou proprioceptive.

**Revendications**

1. Procédé pour obtenir une empreinte corrigée d'un pied afin de fabriquer une semelle correctrice du type dans lequel on appuie le pied (10) sur la paroi supérieure flexible (5a) d'une première enceinte étanche (5c) qui est remplie de matériaux granuleux, qui comporte une paroi supérieure (5a) et une paroi inférieure (5b) flexibles et qui est reliée à un appareil d'aspiration (7) permettant de la mettre en dépression pour rigidifier lesdits matériaux granuleux et pour conserver une empreinte négative du pied (5a), caractérisé en ce que l'on corrige ladite empreinte négative (5a) pendant que ladite enceinte (5c) est connectée audit appareil d'aspiration (7) qui aspire dans ladite enceinte.

2. Procédé selon la revendication 1, caractérisé en ce que l'on obtient, en outre, une empreinte positive (6b) complémentaire de ladite empreinte négative corrigée (5a) en posant sur celle-ci une deuxième enceinte étanche (6c) qui est remplie de matériaux granuleux, qui comporte une paroi supérieure (6a) et une paroi inférieure (6b) flexibles et qui est reliée à un appareil d'aspiration (7) permettant de la mettre en dépression et en posant ledit pied (10) sur ladite paroi supérieure (6a) et on fabrique une semelle correctrice (11) ou un élément de semelle correcteur en utilisant ladite empreinte positive seule ou en combinaison avec ladite empreinte négative corrigée (5a).

3. Procédé selon la revendiction 2, caractérisé en ce que l'on corrige localement en creux ladite empreinte positive en relevant ladite deuxième enceinte et en appuyant localement sur la paroi inférieure de celle-ci pendant que l'enceinte est connectée audit moyen d'aspiration (7); on place à nouveau ladite deuxième enceinte (6c) sur la première enceinte (5c) et on corrige ladite empreinte négative (5a) en appuyant ladite empreinte positive corrigée (6b) contre la face supérieure (5a) de ladite première enceinte (5c) pendant que celle-ci est connectée audit moyen d'aspiration (7) et on fabrique une semelle correctrice (11) ou un élément de semelle correcteur en utilisant ladite empreinte positive corrigée (6b) seule ou en combinaison avec ladite empreinte négative corrigée (5a).

4. Procédé selon l'une quelconque des revendications 1 à 3, caractérisé en ce que l'on obtient une première empreinte négative corrigée en plaçant des éléments correcteurs (8, 9) sous la paroi inférieure (4b) d'une enceinte inférieure (4c) puis en appuyant le pied (10) sur la paroi supérieure (4a) de ladite enceinte inférieure (4c).

5. Procédé selon l'une quelconque des revendications 1 à 4, caractérisé en ce que l'on obtient une empreinte négative corrigée par les opérations suivantes :
- on prend une première empreinte négative de la plante du pied (10) en appuyant celui-ci sur la paroi supérieure souple (4a) d'une enceinte inférieure (4c), remplie d'un matériau granuleux que l'on met ensuite en dépression;
- on corrige éventuellement ladite première empreinte;
- on pose ladite première enceinte(5c) comportant une paroi supérieure (5a) et une paroi inférieure (5b) flexibles sur ladite empreinte négative (4a), on appuie à nouveau le pied (10) sur la paroi supérieure (5a) de ladite première enceinte (5c) et on met ensuite celle-ci en dépression, de sorte que ladite paroi supérieure (5a) de la première enceinte conserve une empreinte négative de la plante du pied qui est corrigée par la présence de ladite première empreinte négative (4a).

6. Procédé selon l'une quelconque des revendications 1 à 5, caractérisé en ce que l'on apporte des corrections en relief auxdites empreintes négative (4a, 5a) en relevant lesdites enceintes (4c, 5c) et en appuyant localement sur la paroi flexible inférieure (4b, 5b) de ladite enceinte inférieure (4c) ou de ladite première enceinte (5c) pendant que celle-ci est connectée sur ledit moyen de mise en dépression (7).

7. Dispositif pour obtenir une empreinte en vue de fabriquer une semelle correctrice du type comportant au moins une enceinte étanche (4c) ayant au moins une paroi flexible (4a) qui est remplie d'un matériau granuleux et qui peut être connectée sur un appareil d'aspiration (7)par l'intermédiaire d'une vanne (4e), caractérisé en ce qu'il comporte au moins deux cadres horizontaux rigides, amovibles (4, 5) et superposables, qui sont équipés chacun d'une paroi supérieure (4a, 5a) et d'une paroi inférieure (4b, 5b) flexibles qui délimitent avec ledit cadre une enceinte étanche (4c, 5c) qui est reliée à un appareil d'aspiration (7) par une canalisation flexible (4d, 5d) munie d'une vanne (4e, 5e) et qui est remplie d'un matériau granuleux.

8. Dispositif selon la revendication 7, caractérisé en ce que lesdites parois supérieures (4a, 5a) sont tendues en travers desdits cadres.

9. Dispositif selon la revendication 8, caractérisé en ce qu'il comporte une enceinte étanche et amovible (6c) qui est superposable au deuxième cadre (5), qui est équipé d'une paroi supérieure (5a) et d'une paroi inférieure (5b) flexibles, qui est remplie d'un matériau granuleux et qui est connectée à un appareil d'aspiration (7) par une canalisation flexible (6d) équipée d'une vanne (6e).

10. Dispositif selon la revendication 7, caractérisé en ce que lesdits premier et deuxième cadres (4, 5) sont reliés à un bâti fixe (3) par des bras (4g, 5g) de longueur différente qui sont articulés audit bâti, de telle sorte qu'il est possible de relever lesdits cadres (4, 5) pour accéder auxdites parois inférieures (4b, 5b) pendant que lesdites enceintes sont connectées audit appareil d'aspiration (7).

Fig. 1

Fig.2

Fig.3

Fig.4

Fig.7

Fig.5

Fig.6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | DE-B-1 234 355  (HULLMANN) <br> * Figures 1-5; colonne 3, ligne 12 - colonne 4, ligne 19 * | 7 | A 61 B  5/10 <br> A 61 F  5/14 |
| A | | 1,4,8 | |
| Y | US-A-3 309 447  (WEGLEY) <br> * Figures 1-4; colonne 2, ligne 17 - colonne 3, ligne 33 * | 7 | |
| A | --- | 1,2,9 | |
| A | FR-A-2 592 282  (DENIS) <br> * Figures 1-10; page 2, ligne 13 - page 4, ligne 23; page 5, ligne 21 - page 17, ligne 13 * | 1-6 | |
| A | --- <br> FR-A-2 440 540  (C.G. DORIS) <br> * Figures 1-5; page 3, ligne 1 - page 4, ligne 40 * <br> ----- | 1,7 | |

**DOMAINES TECHNIQUES RECHERCHES (Int. Cl.4)**

A 61 B
A 61 F

**Le présent rapport a été établi pour toutes les revendications**

| Lieu de la recherche | Date d'achèvement de la recherche | Examinateur |
|---|---|---|
| LA HAYE | 28-12-1988 | CHEN A.H. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................
& : membre de la même famille, document correspondant

EPO FORM 1503 03.82 (P0402)